# EUROPEAN PATENT APPLICATION

(11) **EP 2 799 074 A1**
(43) Date of publication of application: **05.11.2014**
(21) Application number: 14175516.5
(22) Date of filing: 29.11.2010
(51) Int. Cl.: A61K 31/555, A61K 33/26, A61P 7/06, A61K 9/08, A61K 9/10, A61K 9/107, A61K 9/46

(54) **Iron bis-glycinate chelate for use in the oral treatment of anemia in patients with celiac disease**

(30) Priority: 16.12.2009 IT MI20092198
(62) Divisional of application: 10192857.0
(71) Applicant: Laboratori Baldacci S.P.A., 56124 Pisa (IT)
(72) Inventor: Baldacci, Massimo, I-56124 Pisa (IT); Ame', Claudio Aldo, I-10143 Torino (IT)
(74) Representative: Pistolesi, Roberto

(57) **Abstract**

The present invention refers to iron bis-glycinate chelate for use in the oral treatment of anemia in celiac patients.

In particular, in the solid oral forms the iron bis-glycinate chelate according to the invention is administered at an amount varying from 5 to 200 mg per day (corresponding to from I to 40 mg of iron ion), preferably from 10 to 100 mg per day (i.e. corresponding to from 2 to 20 mg of iron ion).

In the semi-solid or liquid forms the iron bis-glycinate chelate according to the invention is administered at an amount varying from 3 to 6 g/100 ml of solution (corresponding to from 6 to 12 mg of iron ion/ml), preferably about 5 g/100 ml of solution (corresponding to 10 mg of iron ion/ml).

## Description

The present invention refers to iron bis-glycinate chelate for use in the oral treatment of anemia in celiac patients.

### Background of the invention

Celiac disease is a small intestine disease with immune-mediated pathogenesis, characterized by a permanent intolerance to protein fractions of wheat, rye, barley and oats. In genetically predisposed subjects, such intolerance causes a mucosal damage which, in turn, leads to atrophy of the small intestine (Farhad Zamani et al., Gluten sensitive enteropathy in patients with iron deficiency anemia of unknown origin World J. Gastroenterol. 2008, 14(48), 7381-7385).

The celiac disease affects both adult subjects and pediatric population.

Actually, celiac disease is one of the most common causes of malabsorption especially at pediatric age, where it is considered one of the chronic diseases most frequently capable of slowing growth.

Generally, clinical manifestations of said disease have a high individual variability and they are conditioned solely partly by the degree of the lesions involving the small intestine (Fernando Bermejo et al. A guide to diagnosis of iron deficiency and iron deficiency anemia in digestive disease World J. Gastroenterol. 2009, 15(37) 4638-4643).

For such reason, there are conditions of extreme heterogeneity ranging from forms with severe malabsorption syndrome to almost asymptomatic forms.

In this connection, it should be observed that the celiac disease causes a modification both in terms of number and form of the intestinal villi, through which the absorption of exogenous and/or nutrient substances occurs.

The celiac disease has various symptoms and each of them is present in celiac subjects at different percentages (Jason W. Harper et al. Anemia in celiac disease is multifactorial in etiology Am. J. Hentatol. 2007, 82, 996-1000).

Some clinical manifestations, or symptoms, present in celiac patients at different degrees of seriousness comprise: weight loss, asthenia, nausea, vomiting, alteration of coagulation, osteopathy with bone pains, profuse mucosity, especially regarding the tongue and neurological disorders of the peripheral nervous system (Farhad Zamani et al., Gluten sensitive enteropathy in patients with iron deficiency anemia of unknown origin World J. Gastroenterol. 2008, 14(48), 7381-7385; Freeman H. et al. Coeliac disease Best. Pract. Res. Clin. Gastroent. 2002, 16(1), 37-49).

Another evident symptom of the celiac disease is represented by diarrhea, whose etiopathogenesis is multifactorial.

One of the causes of diarrhea is malabsorption of foods which leads to an increase of the intraluminal contents of feces which, having a high osmotic capacity, have a greater volume with respect to the physiological conditions. Poor digestion is another cause which leads to the formation of diarrhea. Actually, a reduction of cholecystokinin and secretin secretion is observed in celiac patients which leads to a non-digestion of proteins and lipids.

Furthermore, one of the most significant signs common to about 50% of the patients with celiac disease is represented by sideropenic anemia, observable from the reduction of the hemoglobin, ferritin and serum iron content in the blood following a haemochromocytometric test.

It is known that, in celiac patients, said anemia cannot be treated by oral therapy based on compounds containing iron ions in form of salts, complexes or other substances separated at gastro-enteric level. Actually, according to literature disclosures, oral treatment using compounds containing iron does not cause an increase of the hemoglobin/iron values in the blood of celiac patients (Fernandez-Banares et al. A short review of malabsorption and anemia World J. Gatroenterol. 2009 15(37):4644-4652). Up to now, the consolidated therapy of anemia in celiac patients is constituted by the parenteral administration, in particular intravenous, of iron salts such as, for example, gluconate in therapeutic cycles repeated each 4 or 6 months depending on the seriousness of the anemia. Up to date, only this administration method allows obtaining the therapeutic benefit required for patients affected by said pathology. Up to date, oral treatment based on iron salts has been considered useful solely as a supplement in the stage of maintaining the hemoglobin blood values, subsequent to the parenteral treatment.

The parenteral administration method is very often unappreciated by patients and, particularly disadvantageous considering that it requires the intervention of specialized medical personnel in suitable health facilities or hospitalization of the patient so as to control possible anaphylactic reactions.

Thus, there strongly arises the need to find a therapeutic solution suitable for treating anemia in celiac patients, capable of improving compliance of the patients to the treatment.

### Description of the invention

It was surprisingly found that iron bis-glycinate chelate, administered orally to celiac patients, causes a considerable increase of the levels of hemoglobin, ferritin and serum iron in the blood and it can thus be used efficiently in the treatment of anemia.

This compound, and its structure formula I indicated hereinafter, are well known (Ashmed S.D. The chemistry of iron bis-glycinate chelate, Arch. Latino Am. De Nutr., 2001, 51(1), 7-12; Atkins P.W., Berau J.A. 1992 General Chemistry 2nd ed. Scientific American Books, Wh Freeman New York; Coplin et al. Tolerability of Iron: a comparison of bis-glycino iron II and ferrous sulphate, Clinical Therapeutic, vol. 13, n.5, 606-612, 1991).

The iron bis-glycinate chelate is a rather soluble chelate complex characterized by a metal centre, Fe(II), tetracoordinated by two identical chelating agents (glycine), and corresponds to the following structure formula I:

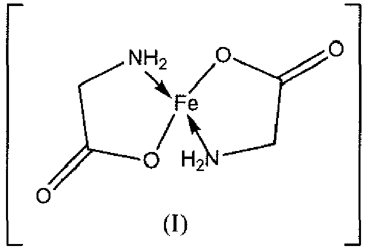

The nitrogen atom dative bond contributes to stabilize the energy of the orbitals of the metal centre and the geometry of the bonding agent by forming a five-atom ring.

This compound is also known for its tolerability, safety and high bioavailability (Jeppsen R. B. Toxicology and safety of Ferrochel and other iron amino acids chelates, Arch. Latino Am. De Nutr., 2001, 51(1), 26-34; Opinion if the scientific Panel on Food additives, Flawouring Processing Aids and materials in contact with Food on a request from the Commision related to: Ferrous bisglycinate as a source of iron for use in the manufacturing of foods and in foods supplements, EFSA Journal, 2006, 299, 1-17). Literature data reveals that such chelate complex has a greater bioavailability with respect to mineral salts, such as for example ferrous sulphate, in that it is absorbed as it is at the intestinal mucosa level and thus it does not undergo any modification in the gastrointestinal system (Pineda O. et al., Effectiveness of iron amino acids chelate on treatment of iron deficiency anaemia in adolescents, Journal of appl. Nutr., Vol. 46, Numbers 1-2, 1994; Pineda O., Effectiveness of treatment of iron deficiency anaemia in infant and young children with ferrous bisglycinate chelate, Nutrition, 2001, 17, 381-384).

The stability of the bond, proven by the fact that the product does not undergo hydrolysis at different pH values of the gastrointestinal section, and the not high molecular weight (204 Daltons) allow maximum absorption when it is administered orally (DeWayne H.A., The absorbtion and metabolism of iron amino acid chelate, Arch. Latino Am. De Nutr., 2001, 51(1), 7-12; Marchetti M et al., Comparison of the rates of vitaminic degradation when mixed with metal sulphates or metal amino acids chelates, J. Food Comp. Anal., 2000, 13, (875-884).

Thus, object of the present invention is iron bis-glycinate chelate for use in the oral treatment of anemia in celiac patients.

According to the present invention, the term "celiac patient" is used to indicate a human being, intended both as an adult subject and as "pediatric population", where the term "pediatric population" indicates the part of the population from birth to eighteen years of age.

The term "anemia" according to the present invention comprises: sideropenic anemia, aplastic anemia, vitamins and/or folates deficiency anemia (such as for example pernicious anemia), chronic diseases anemia (such as for example AIDS, cancer or hepatitis) and hemolytic anemia.

From the therapeutic efficiency results outlined in the present invention (indicated hereinafter in the examples and in the experimental part) obtained by celiac patients suffering from anemia treated with iron bis-glycinate chelate, it can be argued that the chemical/physical characteristics of iron bis-glycinate chelate such as: low molecular weight and the absence of electric charges represent a positive element for absorption through modified intestinal villi observable in most celiac patients.

Contrary to the conventional iron salts taken orally, iron bis-glycinate chelate is not separated into iron ion at a pH interval comprised between 3 and 10.

On the contrary, conventional iron salts taken orally, for example ferrous sulphate, are separated at gastrointestinal level into iron ion and corresponding anion. Fe⁺⁺, having positive charges, is poorly absorbed by the modified intestinal villi.

Preferably, the iron bis-glycinate chelate of the invention is intended to be used for oral treatment of sideropenic anemia in celiac patients.

Sideropenic anemia is a form of anemia characterized by a considerable reduction of hemoglobin in the circulating blood caused by iron deficiency.

The use of iron bis-glycinate chelate for the oral treatment of this type of anemia is thus particularly advantageous in that the iron bis-glycinate chelate according to the present invention can be considered a selection substance to be used in patients with sideropenic anemia, even persistent, and/or in presence of gluten-free diets.

In particular, the iron bis-glycinate according to the present invention, *i.e.* in tablet, preferably in effervescent tablet or in semi-solid or liquid form, is more easily absorbed by modified intestinal villi of a celiac patient with respect to other conventional pharmaceutical forms.

The iron bis-glycinate chelate according to the present invention is thus administered orally, preferably formulated in solid, semi-solid or liquid form, said solid form being selected from among tablet, granulate, micro-granulate or capsule and, said semi-solid or liquid form selected from among suspension or solution.

The iron bis-glycinate chelate of the invention can be formulated associated to at least one sweetener and/or flavor. Preferably, the ratio between said at least one sweetener and/or flavor and the iron bis-glycinate chelate of the invention is equivalent to about 0.56 by weight.

Said at least one sweetener and/or flavor according to the invention can preferably be selected from among acesulfame K, sucralose, sorbitol, sucrose, fructose, orange flavor, lemon flavor, tangerine flavor, caramel flavor or a mixture thereof.

More in particular, the iron bis-glycinate chelate of the present invention is preferably formulated associated to a mixture comprising acesulfame K, sucralose, sorbitol and flavor, where the ratio by weight between acesulfame K : sucralose : sorbitol : flavor is of about 1:0.30-0.50:0.12-0.24:3.0-3.4 respectively, preferably of about 1:0.40:0.18:3.20 by weight.

According to the invention, the iron bis-glycinate chelate can further be formulated associated to further physiologically acceptable excipients and/or additives.

According to the invention, tablet, granulate and micro-granulate can be in coated, non-coated and/or effervescent form, preferably in form of effervescent tablet.

The term "effervescent" according to the present invention is used to indicate a form capable of developing carbon dioxide upon contact with water and/or with the buccal environment, in presence of saliva.

Di, tri-carboxylic acids or a mixture thereof are preferably used to obtain the effervescent form of the invention.

More preferably, the effervescent compositions according to the invention are formulated using dihydrate and monohydrate sodium citrate, sodium carbonate, sodium bicarbonate, potassium bicarbonate, citric acid, tartaric acid, adipic acid, monosodium phosphate, alginic acid, magnesium hydroxycarbonate or a mixture thereof.

In particular, in the oral solid forms the iron bis-glycinate chelate according to the invention is contained at an amount varying from 5 to 200 mg of iron bis-glycinate chelate (corresponding to from 1 to 40 mg of iron ion), preferably from 10 to 100 mg *(i.e.* corresponding to from 2 to 20 mg of iron ion).

Preferably, the iron bis-glycinate chelate is contained at an amount equivalent to about 70 mg for effervescent tablet *(i.e.* corresponding to about 14 mg of iron ion).

A preferred embodiment of the invention comprises the administration of iron bis-glycinate chelate by two effervescent tablets per day containing about 70 mg of iron bis-glycinate chelate *(i.e.* 14 mg of iron ion) for twenty days.

A further embodiment of the invention comprises the administration of iron bis-glycinate chelate by two effervescent tablets per day containing about 70 mg of iron bis-glycinate chelate *(i.e.* 14 mg of iron ion) for twenty days and, an effervescent tablet per day containing about 70 mg of iron bis-glycinate chelate *(i.e.* 14 mg of iron ion) for further forty days ("therapy cycle").

Each therapy cycle according to the invention can be repeated every 3-4 months, for an overall period of about one year.

Another embodiment of the invention comprises the administration of iron bis-glycinate chelate by two effervescent tablets per day, containing about 70 mg of iron bis-glycinate chelate *(i.e.* 14 mg of iron ion), for twenty days, an effervescent tablet per day containing about 70 mg of iron bis-glycinate chelate *(i.e.* 14 mg of iron ion) for further forty days ("therapy cycle"), and a maintenance therapy with 2 effervescent tablets per day containing about 70 mg of iron bis-glycinate chelate *(i.e.* 14 mg of iron ion) for twenty days ("maintenance").

The maintenance according to the present invention can be repeated every 3 months, for an overall period of time of about one year. The term "maintenance" of the invention is used to indicate the continuing of the therapy, with possible increase of the hemoglobin, ferritin and serum iron values.

In the semi-solid or liquid forms the iron bis-glycinate chelate according to the invention is contained at an amount varying from 1 to 10 g/100 ml of solution/suspension (corresponding to from 2 to 20 mg of iron ion/ml), preferably about 5 g/100 ml of solution/suspension (corresponding to 10 mg of iron ion/ml).

Said semi-solid or liquid forms of the invention are preferably administered to the pediatric population, preferably in form of aqueous solution.

Thus, a further embodiment of the invention comprises the administration of iron bis-glycinate chelate to the pediatric population in aqueous solution through a dropper calibrated at 0.5 ml, 0.75 ml and 1 ml to allow adapting the posology according to the age, the weight of the subject and possibly to the seriousness of the celiac disease. According to a further embodiment, the iron bis-glycinate chelate of the invention can be administered associated to one or more further active ingredients.

Further active ingredients according to the present invention can preferably be selected from among vitamins and/or mineral salts.

Said vitamins can preferably be selected from among vitamin B9 (folic acid), vitamin B12 and/or vitamin B6.

Said mineral salts can preferably be selected from among potassium, magnesium, iodine, zinc salts.

The iron bis-glycinate chelate according to the present invention can be administered as a single therapy, or following a conventional therapy both oral and parenteral.

The following examples are intended for a better understanding of the invention, without limiting it in any manner whatsoever.

### EXAMPLES

### Example 1. Formulation in effervescent tablet

| *Active ingredient* | *mg*/*tablet* |
|---|---|
| Iron bis-glycinate chelate: | 70 mg (corresponding to 14 mg of iron ion) |

| *Excipients for effervescence* | |
|---|---|
| Citric acid | 433 mg |
| Sodium bicarbonate | 307 mg |

| *Sweeteners* | |
|---|---|
| Acesulfame K: | 25 mg |
| Sucralose: | 10 mg |
| Sorbitol: | 4.5 mg |

| *Flavar* | |
|---|---|
| Orange flavor: | 80 mg |

The acesulfame K: sucralose: sorbitol: flavor ratio of the effervescent tablet of Example 1 is 1:0.4:0.18:3.20.

The pH of the solution reconstituted by dissolving the effervescent tablet of Example 1 in 150 ml of water is 4.7.

### Examples 2-5: formulation in aqueous solution

**Table A**

| **COMPONENT** | **SOLUTION L1** | **SOLUTION L2** | **SOLUTION L3** | **SOLUTION L4** |
|---|---|---|---|---|
| Iron bis-glycinate chelate | 5.0 g | 5.0 g | 5.0 g | 5.0 g |
| Fructose | 38.5 g | 38.5 g | - | - |
| Sorbitol | 30.8 g | 30.8 g | 30.8 g | 30.8 g |
| Citric acid | 1.5 g | 1.5 g | 1.5 g | 1.5 g |
| Caramel flavor | 1.4 g | - | 1.4 g | - |
| Tangerine flavor | - | 3.0 g | - | 3.0 g |
| Sucrose | - | - | 38.5 g | 38.5 g |
| Acesulfame K | - | - | - | 0.5 g |
| Water | q.s. at 100 ml | q.s. at 100 ml | q.s. at 100 ml | q.s. at 100 ml |
| pH | 4.14 | 4.17 | 4.14 | 4.16 |

### Experimental part

### a) Efficiency comparison between iron bis-glycinate chelate administered orally according to the invention and conventional treatment in the celiac patient.

12 celiac subjects, with sideropenic anemia diagnosed according to the hemoglobin (Hb), ferritin and iron (serum iron) values obtained from the haemochromocytometric test were included in this experiment.

Celiac disease was diagnosed according to the clinical manifestations and histology reports regarding the intestinal mucosa.

All patients were initially treated with ferrous sulphate orally (1 tablet of ferrous sulphate, corresponding to 105 mg of iron ion) for 40 days and/or with sodium ferric gluconate by intravenous administration for a variable period of time according to the seriousness of the anemic status.

Both of these therapies were suspended in that they were inefficient and/or not tolerated. Examples 6-11 indicate the clinical records of some of the celiac patients subjected to treatment using conventional iron salts both through oral (p.o.) and intravenous (i.v.) administration. All patients were diagnosed with celiac disease through bioptic sampling and with serological tests. During the conventional therapy, the diet of said patients was controlled and corrected with gluten-free foods. As evincible from the abovementioned examples 6-11, that follow, regardless of the treatment and diet, the hemoglobin, ferritin and serum iron values remained considerably low.

These patients were then subjected to a therapy using the iron bis-glycinate chelate according to the invention, formulated in the effervescent tablet of example 1.

The hemoglobin (Hb), (Table 1), ferritin (Table 2) and serum iron (Table 3) values taken from each treated patient, both after the conventional therapy (oral and/or intravenous) and after the oral administration according to the present invention (effervescent tablet) were considered for the evaluation.

A therapy cycle corresponds to the treatment for 20 days with two effervescent tablets per day containing about 14 mg of iron ion per tablet and then, an effervescent tablet per day containing 14 mg of iron ion, for further 40 days.

The subsequent maintenance corresponds to the treatment for 20 days with two effervescent tablets per day containing about 14 mg of iron ion per tablet, every 3 months for about one year.

During the treatment, the patients declared to prefer oral treatment with respect to parenteral treatment.

The product did not reveal any adverse effects worth mentioning.

The hemoglobin, ferritin and serum iron blood values were determined after the first treatment cycle and the treatment was continued with repeated cycles according to the previously indicated methods.

As observable from the values indicated in Table 1, 2 and 3, the iron bis-glycinate chelate considerably increases the hemoglobin, ferritin and serum iron value already after I therapy cycle.

Thus, the obtained experimental data shows the efficiency of the oral therapy using iron bis-glycinate chelate of the invention in the treatment of anemia in celiac patients, a condition wherein all compounds administered orally up to date proved inefficient.

### Example 6

### Patient I - Female

### Previous therapies

| Type | Date | Duration | Reason for suspension |
|---|---|---|---|
| Fe sulphate p.o. | 4 years | Few days once in a while | Gastro-intestinal intolerance and ensuing poor efficiency |
| sodium ferric gluconate i.v. (Ferric Fe). | 3 years | 15/18 days cycles every 3-4 months with 1-2 vials/day in 250ml of NaCl | Occurrence of allergy (skin rush) |

At the beginning of the therapy with ferrous sulphate by oral administration the patient has the following values:

| | Hb | Serum iron | Ferritin | Duration of treatment |
|---|---|---|---|---|
| Initial values | 10.2 | 41 | 32 | 40 days |
| Final values | 11.1 | 48 | 38 | |

The treatment cycle was completed, but epistralgia and intestinal disorders occurred.

After other cycles with ferrous sulphate administered orally interrupted due to intestinal intolerance, the patient is subjected to an oral treatment with iron polymaltosate.

| | Hb | Serum iron | Ferritin | Duration of treatment |
|---|---|---|---|---|
| Initial values | 10.6 | 38 | 29 | 20 days |
| Final values | 10.8 | 43 | 25 | |

The treatment cycle is completed and the therapy well tolerated.

During the therapy with some cycles of iron polymaltosate administered orally alternating with those with ferrous sulphate administered orally, the values of Hb were maintained between 9 and 10 gr/dl with occurrence of metrorrhagia and reduction of Hg to 7.7.

Beginning of treatment with ferric iron i.v.

| | | | | | | Hb | Serum iron | Ferritin | Duration of treatment |
|---|---|---|---|---|---|---|---|---|---|
| Initial values | 7.7 | 19 | <5 | 20 days | | | | | |
| Final values | 9.8 | 48 | 30 | - | | | | | |

The treatment cycle was completed with the dosage of 1 vial/day for 10 days, then 2 vials/day.

The patient continued with intravenous administration of ferric iron with cycles every three/four months, maintaining values of Hb between 11 and 13. Following the occurrence of skin rush after infusion of ferric iron, the therapy was suspended.

### Example 7

### Patient II - Female

### Previous therapies

| Type | Data (years) | Duration | Reason for suspension |
|---|---|---|---|
| Fe sulphate p.o. | 5 years, several attempts | Few days | Gastro-intestinal intolerance |
| sodium ferric gluconate i.v. (Ferric Fe). | 2 years, two cycles of 10 vials | 10 days at different periods | Scarce efficiency due to incongruous dosage |

At the beginning of the therapy with ferrous sulphate administered orally the patient has the following values:

| | Hb | Serum iron | Ferritin | Duration of treatment |
|---|---|---|---|---|
| Initial values | 11.6 | 55 | 41 | 8/10 days |
| Final values | 12.3 | 68 | 45 | |

A 40 days treatment cycle has never been completed due to the occurrence, after a few days, of strong epigastralgia.

Subsequently, to the patient other iron formulations were administered orally with the following results:

| | Hb | Serum iron | Ferritin | Few days |
|---|---|---|---|---|
| Initial values | 11.0 | 47 | 29 | |
| Final values | 11.5 | 56 | 33 | |

A 40 days treatment cycle has never been completed due to the occurrence, after a few days, of strong epigastralgia. The values of Hb were maintained at around 11 gr/dl.

Beginning of treatment with ferric iron by intravenous administration:

| | Hb | Serum iron | Ferritin | Duration of treatment |
|---|---|---|---|---|
| Initial values | 10.4 | 40 | 31 | Few days |
| Final values | 10.8 | 49 | 33 | |

The treatment cycle was not completed due to fear of the infusion needle and work difficulty of the patient.

### Example 8

### Patient III - Male - Vegetarian patient

### Previous therapies

| Type | Date (years) | Duration | Reason for suspension |
|---|---|---|---|
| Fe sulphate p.o. | 5 years | One month cycles once in a while | Scarce efficiency |
| sodium ferric gluconate i.v. (Ferric Fe). | Never administered | - | i.v. treatment consent denied by patient |

At the beginning of the therapy with ferrous sulphate administered orally the patient has the following values:

| | Hb | Serum iron | Ferritin | Duration of treatment |
|---|---|---|---|---|
| Initial values | 9.1 | 32 | 19 | 40 days |
| Final values | 10 | 38 | 27 | |

Treatment cycle completed with occurrence of strong epigastralgia and intestinal disorders.

The patient never accepted the disease, neither took suitable iron dosages nor followed the diet correctly.

### Example 9

### Patient IV - Female

### Previous therapies

| Type | Date (years) | Duration | Reason for suspension |
|---|---|---|---|
| Fe sulphate p.o. | Never taken | - | - |
| sodium ferric gluconate i.v. (Ferric Fe). | 3 years | 15/20 days cycles with 1 vial i.v. | Scarce efficiency at the beginning and then intolerance occurred. |

Ferrous sulphate administered orally never taken by the patient due to the serious gastrointestinal disorders (gastric ulcers and irritable colon).

Beginning of the therapy with ferric iron by intravenous administration:

| | Hb | Serum iron | Ferritin | Duration of treatment |
|---|---|---|---|---|
| Initial values | 9.7 | 39 | 21 | 20 days |
| Final values | 10.6 | 54 | 42 | |

Treatment cycle completed with dosage of 1 vial/day for 10 days, then 2 vials/day.

The patient continued with iron by intravenous administration with cycles every 3/4 months, with values of Hb at around 11 gr/dl. Therapy suspended due to occurrence of skin rush and asthmatic crisis after infusion of iron by intravenous administration.

### Example 10

### Patient V - Female

### Previous therapies

| Type | Date (years) | Duration | Reason for suspension |
|---|---|---|---|
| Fe sulphate p.o. | - | Several attempts of 30/40 days | Scarce efficiency and occurrence of gastrointestinal disorders. |
| sodium ferric | Only one cycle | Few days | Suspended due to occurrence |
| gluconate i.v. (Ferric Fe). | | | of allergy |

After several therapy cycles with ferrous sulphate administered orally the patient has the following values:

| | Hb | Serum iron | Ferritin | Duration of treatment |
|---|---|---|---|---|
| Initial values | 8.4 | 22 | <5 | 40 days |
| Final values | 9.7 | 46 | 21 | |

Due to the intestinal disorders observed on the patient, the therapy with iron by intravenous administration was tried. Suspended after three infusions due to the occurrence of allergic reaction.

### Example 11

### Patient VI - Male

### Previous therapies

| Type | Date (years) | Duration | Reason for suspension |
|---|---|---|---|
| Fe sulphate p.o. | 1 year | Few days | Intestinal disorders (diarrhea). |
| sodium ferric gluconate i.v. (Ferric Fe). | 2 years | 3 cycles of 20 days with 1 vial i.v. in 250ml of saline solution | Occurrence of intolerance at the third cycle (phlebopathy) |

Therapy with ferrous sulphate administered orally suspended due to the occurrence after a few days of profuse diarrhea. Beginning of the therapy with iron by intravenous administration.

| | Hb | Serum iron | Ferritin | Duration of treatment |
|---|---|---|---|---|
| Initial values | 10.2 | 41 | 35 | 20 days |
| Final values | 12.6 | 68 | 81 | |

Occurrence of phlebopathy during infusion after the third therapy cycle.

**Table 1: Hemoglobin values after oral treatment with ferrous sulphate and/or intravenous treatment with sodium ferric gluconate with respect to oral treatment with the effervescent tablet of example 1.**

| **Pt/ Gender** | **Initial therapy** | **Reason for suspension** | **Hb values (g/100 ml)** | **Efferv. tab therapy* 1^{st} cycle** | **Hb values (g/100 ml)** | **Maintenance efferv. tab.**** | **Hb values (g/100 ml)** |
|---|---|---|---|---|---|---|---|
| 1/F | 1) Fe sulphate p.o. | 1) Inefficiency | 9.1 | Iron bis-glycinate chelate | 11.8 | Iron bis-glycinate chelate | 12.3 |
| | 2) sodium ferric gluconate i.v. | 2) Intolerance | | | | | |
| 2/F | 1) Fe sulphate p.o. | 1) Inefficiency | 9.9 | Iron bis-glycinate chelate | 12.3 | Iron bis-glycinate chelate | 12.9 |
| | 2) sodium ferric gluconate i.v. | 2) Intolerance | | | | | |
| 3/F | 1) Fe sulphate p.o. | 1) Inefficiency | 10.3 | Iron bis-glycinate chelate | 12.7 | Iron bis-glycinate chelate | 13.1 |
| | 2) sodium ferric gluconate i.v. | 2) Intolerance and scarce efficiency | | | | | |
| 4/M ∘ | Fe sulphate p.o. | Inefficiency | 8.9 | Iron bis-glycinate chelate | 11.9 | Iron bis-glycinate chelate | 12.7 |
| 5/F | sodium ferric gluconate i.v. | Intolerance and inefficiency | 10.4 | Iron bis-glycinate chelate | 12.1 | Iron bis-glycinate chelate | 13.4 |
| 6/F | 1) Fe sulphate p.o. | 1) Inefficiency | 8.6 | Iron bis-glycinate chelate | 10.0 | Iron bis-glycinate chelate | 12.1 |
| | 2) sodium ferric gluconate i.v. | 2) Intolerance | | | | | |
| 7/F | Fe sulphate p.o. | Inefficient and not tolerated by the patient | 11.1 | Iron bis-glycinate chelate | 12.5 | Iron bis-glycinate chelate | 13.6 |
| 8/F | 1) Fe sulphate p.o. | 1) Inefficiency | 10.9 | Iron bis-glycinate chelate | 12.0 | Iron bis-glycinate chelate | 12.9 |
| | 2) sodium ferric gluconate i.v. | 2) Intolerance | | | | | |
| 9/M | 1) Fe sulphate p.o. | 1) Inefficiency | 11.8 | Iron bis-glycinate chelate | 13.1 | Iron bis-glycinate chelate | 14.3 |
| | 2) sodium ferric gluconate i.v. | 2) Intolerance | | | | | |
| 10/F | 11) Fe sulphate p.o. | 1) Inefficiency | 10.2 | Iron bis-glycinate chelate | 11.7 | Iron bis-glycinate chelate | 13.4 |
| | 2) sodium ferric gluconate i.v. | 2) Intolerance | | | | | |
| 11/F | 1) Fe sulphate p.o. | 1) Inefficiency | 9.9 | Iron bis-glycinate chelate | 12.0 | Iron bis-glycinate chelate | 13.2 |
| | 2) sodium ferric gluconate i.v. | 2) Intolerance | | | | | |
| 12/M | 1) Fe sulphate p.o. | 1) Inefficiency | 11.1 | Iron bis-glycinate chelate | 12.8 | Iron bis-glycinate chelate | 14.0 |
| | 2) sodium ferric gluconate i.v. | 2) Intolerance | | | | | |

| | | | | | | | |
|---|---|---|---|---|---|---|---|
| * two effervescent tablets per day for 20 days, then one tablet per day for 40 days. ** two effervescent tablets per day for 20 days every 3 months for about one year. ∘ The patient follows a vegetarian diet. | | | | | | | |

**Table 2 Values of ferritin following oral treatment with ferrous sulphate and/or intravenous treatment with sodium ferric gluconate with respect to oral treatment with the effervescent tablet of example 1.**

| **Pt/ Gender** | **Initial therapy** | **Reason for suspension** | **Values of Ferritin (ng/ml)** | **Efferv. tab therapy* 1^{st} cycle** | **Values of Ferritin (ng/ml)** | **Maintenance efferv tab.**** | **Ferritin values (ng/ml)** |
|---|---|---|---|---|---|---|---|
| 1/F | 1) Fe sulphate p.o. | 1) Inefficiency | 20 | Iron bis-glycinate chelate | 121 | Iron bis-glycinate chelate | 147 |
| | 2) sodium ferric gluconate i.v. | 2) Intolerance | | | | | |
| 2/F | 1) Fe sulphate p.o. | 1) Inefficiency | 28 | Iron bis-glycinate chelate | 101 | Iron bis-glycinate chelate | 146 |
| | 2) sodium ferric gluconate i.v. | 2) Intolerance | | | | | |
| 3/F | 1) Fe sulphate p.o. | 1) Inefficiency | 18 | Iron bis-glycinate chelate | 111 | Iron bis-glycinate chelate | 177 |
| | 2) sodium ferric gluconate i.v. | 2) Intolerance and scarce efficiency | | | | | |
| 4/M ∘ | Fe sulphate p.o. | Inefficiency | <5 | Iron bis-glycinate chelate | 43 | Iron bis-glycinate chelate | 92 |
| 5/F | sodium ferric gluconate i.v. | Intolerance and inefficiency | 22 | Iron bis-glycinate chelate | 100 | Iron bis-glycinate chelate | 143 |
| 6/F | 1) Fe sulphate p.o. | 1) Inefficiency | 7 | Iron bis-glycinate chelate | 63 | Iron bis-glycinate chelate | 79 |
| | 2) sodium ferric gluconate i.v. | 2) Intolerance | | | | | |
| 7/F | Fe sulphate p.o. | Inefficient and not tolerated by the patient | 23 | Iron bis-glycinate chelate | 80 | Iron bis-glycinate chelate | 193 |
| 8/F | 1) Fe sulphate p.o. | 1) Inefficiency | 20 | Iron bis-glycinate chelate | 96 | Iron bis-glycinate chelate | 134 |
| | 2) sodium ferric gluconate i.v. | 2) Intolerance | | | | | |
| 9/M | 1) Fe sulphate p.o. | 1) Inefficiency | 27 | Iron bis-glycinate chelate | 119 | Iron bis-glycinate chelate | 206 |
| | 2) sodium ferric gluconate i.v. | 2) Intolerance | | | | | |
| 10/F | 1) Fe sulphate p.o. | 1) Inefficiency | 14 | Iron bis-glycinate chelate | 54 | Iron bis-glycinate chelate | 127 |
| | 2) sodium ferric gluconate i.v. | 2) Intolerance | | | | | |
| 11/F | 1) Fe sulphate p.o. | 1) Inefficiency | 12 | Iron bis-glycinate chelate | 78 | Iron bis-glycinate chelate | 151 |
| | 2) sodium ferric gluconate i.v. | 2) Intolerance | | | | | |
| 12/M | 1) Fe sulphate p.o. | 1) Inefficiency | 24 | Iron bis-glycinate chelate | 67 | Iron bis-glycinate chelate | 200 |
| | 2) sodium ferric gluconate i.v. | 2) Intolerance | | | | | |

| | | | | | | | |
|---|---|---|---|---|---|---|---|
| * two effervescent tablets per day for 20 days, then one tablet per day for 40 days. ** two effervescent tablets per day per 20 days every 3 months for about one year. ∘ The patient follows a vegetarian diet. | | | | | | | |

**Table 3 Values of serum iron following oral treatment with ferrous sulphate and/or intravenous treatment with sodium ferric gluconate with respect to oral treatment with the effervescent tablet of example 1.**

| **Pt/ Gender** | **Initial therapy** | **Reason for suspension** | **Serum iron values (mcg/dl)** | **Efferv. tab Therapy* 1^{st} cycle** | **Values of serum ion (mcg/dl)** | **Maintenance Efferv tab**.** | **Serum iron values (mcg/dl)** |
|---|---|---|---|---|---|---|---|
| 1/F | 1) Fe sulphate p.o. | 1) Inefficiency | 27 | Iron bis-glycinate | 64 | Iron bis-glycinate chelate | 71 |
| | 2) sodium ferric gluconate i.v. | 2) Intolerance | | chelate | | | |
| 2/F | 1) Fe sulphate p.o. | 1) Inefficiency | 31 | Iron bis-glycinate | 70 | Iron bis-glycinate | 77 |
| | 2) sodium ferric gluconate i.v. | 2) Intolerance | | chelate | | chelate | |
| 3/F | 1) Fe sulphate p.o. | 1) Inefficiency | 39 | Iron bis-glycinate chelate | 92 | Iron bis-glycinate chelate | 84 |
| | 2) sodium ferric gluconate i.v. | 2) Intolerance and scarce efficiency | | | | | |
| 4/M ∘ | Fe sulphate p.o. | Inefficiency | 16 | Iron bis-glycinate chelate | 56 | Iron bis-glycinate chelate | 68 |
| 5/F | sodium ferric gluconate i.v. | Intolerance and inefficiency | 30 | Iron bis-glycinate chelate | 65 | Iron bis-glycinate chelate | 78 |
| 6/F | 1) Fe sulphate p.o. | 1) Inefficiency | 19 | Iron bis-glycinate chelate | 44 | Iron bis-glycinate chelate | 51 |
| | 2) sodium ferric gluconate i.v. | 2) Intolerance | | | | | |
| 7/F | Fe sulphate p.o. | Inefficient and not tolerated by the patient | 34 | Iron bis-glycinate chelate | 62 | Iron bis-glycinate chelate | 67 |
| 8/F | 1) Fe sulphate p.o. | 1) Inefficiency | 29 | Iron bis-glycinate chelate | 48 | Iron bis-glycinate chelate | 51 |
| | 2) sodium ferric gluconate i.v. | 2) Intolerance | | | | | |
| 9/M | 1) Fe sulphate p.o. | 1) Inefficiency | 32 | Iron bis-glycinate chelate | 73 | Iron bis-glycinate chelate | 87 |
| | 2) sodium ferric gluconate i.v. | 2) Intolerance | | | | | |
| 10/F | 1) Fe sulphate p.o. | 1) Inefficiency | 33 | Iron bis-glycinate chelate | 41 | Iron bis-glycinate chelate | 80 |
| | 2) sodium ferric gluconate i.v. | 2) Intolerance | | | | | |
| 11/F | 1) Fe sulphate p.o. | 1) Inefficiency | 25 | Iron bis-glycinate chelate | 57 | Iron bis-glycinate chelate | 93 |
| | 2) sodium ferric gluconate i.v. | 2) Intolerance | | | | | |
| 12/M | 1) Fe sulphate p.o. | 1) Inefficiency | 32 | Iron bis-glycinate chelate | 61 | Iron bis-glycinate chelate | 103 |
| | 2) sodium ferric gluconate i.v. | 2) Intolerance | | | | | |

| | | | | | | | |
|---|---|---|---|---|---|---|---|
| * two effervescent tablets per day for 20 days, then one tablet per day for 40 days. ** two effervescent tablets per day for 20 days every 3 months for about one year. ° The patient follows a vegetarian diet. | | | | | | | |

### Conclusions

Thus, as evincible from the examples and tables indicated above, only the oral therapy with iron bis-glycinate chelate according to the present invention allowed a considerable increase of the examined blood parameters (serum iron, ferritin, hemoglobin) in celiac patients suffering from anemia and eliminating the adverse effects (gastrointestinal or others) due to the intake of conventional iron salts both by oral and intravenous administration.

When the therapy with iron bis-glycinate chelate according to the present invention was undertaken it could be assumed, according to the known data, that the product could reveal better tolerance, but such significant efficiency results were totally unexpected.

## Claims

1. Iron bis-glycinate chelate for use in the oral treatment of anemia in patients affected by celiac disease in form of a semi-solid or liquid form.

2. Iron bis-glycinate chelate according to claim 1, wherein said semi-solid or liquid form is selected from among suspension or solution.

3. Iron bis-glycinate chelate according to claim 2, wherein said semi-solid or liquid form contains an amount varying from 1 to 10 g/100 ml of solution/suspension, preferably about 5 g/100 ml of solution/suspension.

4. Iron bis-glycinate chelate according to any one of the preceding claims, wherein said semi-solid or liquid form contains an amount varying from 2 to 20 mg of iron ion/ml, preferably about 10 mg of iron ion/ml.

5. Iron bis-glycinate chelate according to any one of the preceding claims, wherein said iron bis-glycinate chelate is formulated associated to at least one sweetener and/or a flavor.

6. Iron bis-glycinate chelate according to any one of the preceding claims, wherein said iron bis-glycinate chelate is administered associated to at least one further active ingredient.

7. Iron bis-glycinate chelate according to claim 1, **characterized in that** said anemia is sideropenic anemia.

8. Iron bis-glycinate chelate according to any one of the preceding claims, wherein said iron bis-glycinate chelate is administered to the pediatric population, preferably in form of an aqueous solution.

9. Formulation in form of a semi-solid or liquid form containing iron bis-glycinate chelate in an amount varying from 1 to 10 g/100 ml of solution /suspension.

10. Formulation according to claim 9, wherein said amount of iron bis-glycinate chelate is about 5 g/100 ml of solution/suspension.

11. Formulation according to any one of claims 9-10 in form of an aqueous solution.
